# EUROPEAN PATENT SPECIFICATION

(11) **EP 0 290 446 B1**
(45) Date of publication and mention of the grant of the patent: **02.02.1994**
(21) Application number: 87900757.3
(22) Date of filing: 23.12.1986
(51) Int. Cl.: C12N 15/45, A61K 39/155

(54) **VACCINES FOR HUMAN RESPIRATORY VIRUS**
IMPFSTOFFE GEGEN MENSCHLICHE RESPIRATORISCHE VIREN
VACCINS CONTRE LE VIRUS RESPIRATOIRE HUMAIN

(30) Priority: 14.01.1986 US 818740
(43) Date of publication of application: 17.11.1988
(73) Proprietor: UNIVERSITY OF NORTH CAROLINA AT CHAPEL HILL, Chapel Hill, North Carolina 27514 (US)
(72) Inventor: WERTZ, Gail, W., Chapel Hill, NC 27514 (US); COLLINS, Peter, L., Kensington, MD 20895 (US)
(74) Representative: Perry, Robert Edward
(86) International application number: US8602756
(87) International publication number: WO8704185

(56) References cited:
- Proceedings of the natioal Academy of Sciences of the USA, vol. 81, December 1984, P.L. Collins et al "Nucleotide sequence of the gene encoding the fusion (F) glycoprotein of human respiratory syncytial virus", pages 7683-7687
- Chemical abstracts, vol. 104, n 21, 26 May 1986, (Columbus, Ohio, US) M.D. Summers et al "Genetic engineering of the genome of the autographa californica nuclear polyhedrosis virus", see page 125, abstract 18075w & Banbury Rep. 1985, 22 (Genet. Altered Viruses Environ.), 319-39
- Proceedings of the National Academy of Sciences of the USA, vol. 82, June 1985, G.W. Wertz et al "Nucleotide sequence of the G protein gene of human respiratory syncytial virus reveals an unusual type of viral membrane protein", pages 4075-4079
- Journal of Virology, vol. 54, n 1, April 1985, American Society for Microbiology, P.L. Collins et al "The envelope-associated 22K protein of human respiratory syncytial virus : Nucleotide sequence of the mRNA and a related polytranscript", pages 65-71
- Virology, vol. 146, n 1, January 1985, Academic Press, inc. P.L. Collins et al "Correct sequence for the major nucleocapsid protein mRNA of respiratory syncytial virus", pages 69-77

## Description

### BACKGROUND OF THE INVENTION

### Field of the Invention.

This invention discloses compositions of DNA and protein that are useful for preparing vaccines against human respiratory syncytial virus [HRSV]. The proteins include the native structural viral proteins and immunogenic fragments thereof. The DNA compositions include structural genes coding for these proteins and expression and replication plasmids containing the structural genes. Host cells transformed with the above DNA compositions are also disclosed herein. Lastly vaccines comprised of the native structural viral proteins and their immunogenic derivatives are disclosed as well as methods for protecting humans by inoculation with said vaccines. This invention was made with Government support under At-12464 awarded by the National Institute of Health. The Government has certain rights in the invention.

### Background.

HRSV was first discovered in 1956 and is worldwide in distribution. It is an important cause of upper and lower respiratory tract disease causing illness in infants and young children. In infants this severe illness often requires hospitalization. About 30 percent of hospitalized young children with acute respiratory disease have respiratory syncytial virus infection. In older children and adults the disease is milder. Infections with respiratory syncytial virus are referable to all segments of the respiratory tract, are usually associated with fever, cough, runny nose, and fatigue, and are diagnosed clinically as bronchitis, bronchiolitis, pneumonia, croup, or viral infection. In older children and adults the virus is generally limited to replication in the upper respiratory tract. Infants may be more severely involved when the virus extends into the lungs. Lung damage can be permanent.

Primary infection with respiratory syncytial virus occurs early in life, usually before 4 years of age. Among children, illness caused by this virus tends to occur at least once each year in rather sharply defined outbreaks of several months duration. Epidemics are sharply circumscribed, generally for 3 to 5 months. In family studies, children in early school years frequently introduce the virus into the home, infecting younger members of the family more severely than other family members. The clinical consequence of infection is most severe on first experience and becomes milder in older individuals who are immunologically experienced.

The effects of respiratory syncytial virus can range from inapparent infection to severe pneumonia and death. Inflammation of the respiratory track is responsible for most symptoms. Complete recovery in most cases occurs in one to three weeks with the production of antibody which appears to persist throughout life. In the United States about 30 percent of 1-year old infants and 95 percent of 5-year old children have circulating respiratory syncytial virus antibody. Reinfections in older infants, children, and adults with antibody are mostly mild upper respiratory illnesses in the form of colds.

With exception of the present invention, there are no effective vaccines to combat HRSV.

### Description of the Prior Art

Although low yields of virus in cell culture have hindered HRSV research, the virus has been well studied. HRSV is a paramyxovirus containing a single negative strand of RNA which is transcribed into 10 predominantly monocistronic messengers. The messengers have been isolated and translated in vitro. The products have been characterized by gel electrophoresis, peptide mapping and immuno-precipitation as being similar to structural proteins isolated from virions. The structural proteins include a major nucleocapsid protein (N; MW ca. 42,000), a nucleocapsid phosphoprotein (P; MW ca. 34,000), a large nucleocapsid protein (L; MW ca. 200,000), an envelope matrix protein (M; MW ca. 26,000), a matrix glycoprotein (ca. 22,000) and two envelope glycoproteins, the fusion glycoprotein (F; MW ca. 68,000 to 70,000) and a second, methioninepoor glycoprotein (G; MW ca. 84,000 to 90,000). In addition, a virally encoded protein of about 9,500 daltons and other small proteins are known to be present in infected cells. Collins, P.L., et al., Identification of a tenth mRNA of RSV and assignment of polypeptides to the 10 viral genes-,J. of Virol. 49:572 -578 (1984) and references cited therein. Although the structural proteins of HRSV have been isolated, their amino acid sequences are not known.

Multiple attempts have been made to obtain an effective vaccine against HRSV. Friedewald et al., Journal of the American Medical Association, Vol. 204, 20 May 1968, pp. 690-694 describe the propagation of respiratory syncytial virus in bovine embryonic kidney tissue culture. Virus grown at 34°C or 28°C did not decrease in infectivity or virulence. HRSV grown at 26°C, while associated with a decrease in infectivity for adults, could not be considered for use in prevention of infection in adults since the virus had limited infectivity and was poorly immunogenic.

Kim et al., Pediatrics, Vol. 48, November 1971, pp. 745-755, disclose that inactivated respiratory syncytial virus vaccine prepared from virus grown at 26°C stimulated the development of high levels of serum antibody in infants and children from 6 months to 13 years in age but did not prevent infection.

McIntosh et al., Pediatric Research, Vol. 8, 1974, pp. 689-696, discuss two experimental live respiratory syncytial virus vaccines, one prepared from virus grown at 26°C. and the other, prepared from a temperature sensitive mutant which grew well at 32°C and not at all at 37°C. or higher. The first vaccine was unsatisfactory as it did not protect against infection when the interval between vaccination and challenge was greater than 4 months. The second vaccine was also unsatisfactory in that it apparently lost its temperature sensitivity in some vaccinees.

Craighead, Journal of Infectious Diseases, Vol. 131, June 1975, pp. 749-753, discusses tests conducted in 1966 wherein several groups of investigators tested in infants and young children a formaldehyde-treated, alum-precipitated virus grown in tissue culture. Upon subsequent exposure to wild virus the vaccine recipients exhibited an accentuated pattern of respiratory tract disease. Craighead concludes that immunization with formaldehyde treated virus enhanced the severity of the disease.

Wright et al., Journal of Pediatrics, Vol. 88, June 1976, pp. 931-936, describe the evaluation in infants of a temperature sensitive live attenuated respiratory syncytial vaccine. While this vaccine when administered at a dosage level sufficiently high to infect all seronegative infants caused mild upper respiratory illness, lowering the dose did not achieve an acceptable level of infectivity. The virus was also genetically unstable as there was evidence of loss of temperature sensitivity in one vaccinee. There was no evidence for potentiation of natural illness with this vaccine and reinfection occurred among vaccinees.

US-A-4122167 and US-A-4145252 describe a method for attenuating virions by serial passage through human diploid lung fibroblasts. US-A-4517304 discloses a method for producing immunogenically-active HRSV proteins upon the cell membranes of susceptible cells grown in culture. These cells are then injected into a host, to elicit an immune response.

The references described above attempt to create a vaccine by injection of virions comprised of both protein and nucleic acid or by injection of undefined compositions of virus proteins attached to the cell membranes of host cells. None of the above work has resulted in an effective vaccine; see Raeburn, The Houdini Virus, Science 85, Vol. 6:52-57 (Dec. 1985).

Collins et al, Proc. Natl. Acad. Sci. USA 81:7683-7687 (December 1984), discloses the gene sequence for the F glycoprotein. Wertz et al, Proc. Natl. Acad. Sci. USA 82:4075-4079 (June 1985), discloses the gene sequence for the G glycoprotein.

### SUMMARY OF THE INVENTION

According to the present invention, a human respiratory syncytial virus vaccine comprises glycosylated F protein, glycosylated G protein or a derivative thereof having sufficient antigenic capacity to produce effective immunologic protection in a patient exposed to virulent HRSV, free of other virus-synthesised protein.

### DESCRIPTION OF THE INVENTION

Vaccines of the invention can be tailored to contain any proportion of the structural proteins that will best afford immuno-protection. From a safety perspective this invention avoids the exposure of young children to intact HRSV virions, either inactivated or attenuated, and to viral nucleic acid. By avoiding the injection of a complete virion, the vaccines disclosed herein need not be treated with a fixative such as formaldehyde which has been shown to result in the development of ineffective antibodies and in the subsequent increased susceptibility of the host/patient when exposed to virulent HRSV.

It has been demonstrated that the vaccinia virus expression system is useful for expressing the G and F glycoproteins of HRSV; see Ball et al, P.N.A.S. USA 83:246-250 (1986), and Olmsted, P.N.A.S. USA 83:7462-7466 (1986). These two glycoproteins also induce immunoprotection in mammals against a live HRSV virus challenge; see Stott et al, J. Virology 67:607-613 (1986), and also P.N.A.S. USA 83:246-250 (1986). The methodology and results of these references are all incorporated by reference herein.

The specific sequence and base numbering positions for the disclosed proteins of HRSV strain A₂ are illustrated in Charts 1 and 2. Charts 1 and 2 respectively contain the nucleic acid sequences for the F and G HRSV structural proteins.

It is anticipated that protein from the A₂ strain will induce cross-protection against other strains of HRSV; however, it is possible that maximum protection will involve immunization with a mixture of proteins from various strains.

### A. General Methods

Generally, the nomenclature and general laboratory procedures required in this application can be found in Maniatis, T. et al., Molecular Cloning A laboratory Manual, Cold Spring Harbor laboratory, Cold Spring Harbor, New York, 1982. The manual is hereinafter referred to as Maniatis.

All E. coli strains are grown on Luria broth (LB) with glucose, Difco's Antibiotic Medium #2 and M9 medium supplemented with glucose and acid-hydrolyzed casein amino acids. Strains with resistance to antibiotics were maintained at the drug concentrations described in Maniatis. Transformations were performed according to the method described by Rowekamp, W. and Firtel, R.A., Dev. Biol., 79:409-418 (1980).

All enzymes were used according to the manufacturer's instructions. Transformants were analyzed by colony hybridization as described in Grunstein, M. and Wallis, J., Methods in Enzymology, 68:379-388.

After hybridization, the probes are removed and saved, and the filters are washed in 0.1% SDS, 0.2x SSC for a total of 3 hours with 5 changes of 400 ml each. Filters are thoroughly air dried, mounted, and autoradiographed using Kodak X-OMAT AR film and Dupont Cronex Lightnening Plus intensifying screens for 16 hours at -70° C.

For sequencing of plasmids, purified plasmid DNA is prepared according to the methods described in Maniatis. End-labeled DNA fragments are prepared and analyzed by the chemical sequencing methods of Maxam and Gilbert with modifications described by Collins, P.L. and Wertz, G.W., J. Virol. 54:65-71 (1985).

Nucleotide sizes are given in either kilobases (kb) or basepairs (bp). These are estimates derived from agarose gel electrophoresis.

### B. HRSV cDNA

The first step in obtaining expression of HRSV proteins is to obtain the DNA sequence coding for the protein from cDNA clones. This sequence is then cloned into an expression plasmid which is capable of directing transcription of the gene and allowing efficient translation of the transcript.

The library method for obtaining cDNA encoding HRSV proteins has been described generally in Maniatis, T., Fritsh, E. F., and Sambrook, J. (1982). Molecular Cloning, A laboratory Manual. Cold Spring Harbor laboratory, New York.and specifically in Collins, P.L. and Wertz, G.W., cDNA cloning and transcriptional mapping of nine polyadenylated RNAs encoded by the genome of HRSV, Proc. Natl.-Acad. USA 80:3208-3212 (1983).

Clones are prepared by inserting the cDNA into PstI cleaved pBR322 to which homopolymer tracts of dGTP have been enzymatically added to the 3'ends at the cleavage site. Homopolymer tracts of dCTP are enzymatically added to the 3' termini of the cDNA molecules according to the methods described by Maniatis. Ideally, 10-30 residues of dCTP or dGTP should be added to maximize cloning efficiency. The cDNA and plasmid are annealed together and transformed into E. coli. The clones containing full length HRSV cDNA are detected by probes of labeled viral cDNA or oligonucleotides complementary to portions of the sequences illustrated in Charts 12-16, followed by restriction enzyme analysis and DNA sequencing.

Oligonucleotides are chemically synthesized according to the solid phase phosphoramidite triester method first described by Beaucage S.L. and Caruthers, M.H. Tetrahedron Letts. 22(20):1859-1862 (1981) using an automated synthesizer, as described in Needham-VanDevanter, D.R., et al., Nucleic Acids Res., 12:6159-6168 (1984). Purification of oligonucleotides is by either native acrylamide gel electrophoresis or by anion-exchange HPLC as described in Pearson, J.D. and Regnier, F.E., J. Chrom., 255:137-149 (1983).

The sequence of the synthetic oligonucleotides can be verified using the chemical degradation method of Maxam, A.M. and Gilbert, W., Grossman, L. and Moldave, D., eds., Academic Press, New York, Methods in Enzymology, 65:499-560 (1980).

### C. Expression in E. coli.

To obtain high level expression of a cloned gene, e.g., the HRSV protein cDNA, in a prokaryotic system, it is essential to construct expression vectors which contain, at the minimum, a strong promoter to direct mRNA transcription, a ribosome binding site for translational initiation, and a transcription terminator. Examples of regulatory regions suitable for this purpose are the promoter and operator region of the E. coli tryptophan biosynthetic pathway as described by Yanofsky, C., Kelley, R.L. and Horn, V., J. Bacteriol., 158:1018-1024 (1984) and the leftward promoter of phage lambda (P_{L}) as described by Herskowitz, I. and Hagen. D., Ann. Rev. Genet., 14:399-445 (1980).

The HRSV-like proteins produced in E. coli will not fold properly due to the presence of cysteine residues and to the lack of suitable post-translational modifications. During purification from E. coli, the expressed proteins must first be denatured and then renatured. This can be accomplished by solubilizing the E. coli produced proteins in guanidine HCl and reducing all the cysteine residues with β-mercaptoethanol. The protein is then renatured either by slow dialysis or by gel filtration. US Patent No. 4,511,503.

Detection of HRSV-like proteins is achieved by methods known in the art such as radioimmunoassays, or Western blotting techniques or immunoprecipitation. Purification from E. coli can be achieved following procedures described in US Patent No. 4,511,503.

### D. Expression of HRSV-like proteins in Yeast.

Expression of heterologous proteins in yeast is well known and described. Methods in Yeast Genetics, Sherman, F., et al., Cold Spring Harbor laboratory, (1982) is a well recognized work describing the various methods used to produce HRSV-like proteins in yeast.

For high level expression of a gene in yeast, it is essential to connect the gene to a strong promoter system as in the prokaryote and to also provide efficient transcription termination/polyadenylation sequences from a yeast gene. Examples of useful promoters include GAL1,10 (Johnston M., and Davis, R.W., Mol. and Cell. Biol., 4:-1440-48, 1984), ADH2 (Russell, D., et al., J. Biol. Chem. 258:-2674-2682, 1983), PHO5 (EMBOJ. 6:675-680, 1982), and MFα1. A multicopy plasmid with a selective marker such as Lue-2, URA-3, Trp-1, and His-3 is also desirable. The MFα1 promoter is preferred. The MFα1 promoter, in a host of the a mating-type is constitutive, but is off in diploids or cells with the a mating-type. It can, however, be regulated by raising or lowering temperature in hosts which have a ts mutation at one of the SIR loci. The effect of such a mutation at 35°C on an α type cell is to turn on the normally silent gene coding for the a mating-type. The expression of the silent a mating-type gene, in turn, turns off the MFα1 promoter. Lowering the temperature of growth to 27°C reverses the whole process, i.e., turns the a mating-type off and turns the MFα1 on (Herskowitz, I. & Oshima, Y. (1982) in The molecular biology of the yeast saccharomyces, (eds. Strathern, J.N., Jones, E.W., & Broach, J.R., Cold Spring Harbor Lab., Cold Spring Harbor, NY, pp 181-209).

The polyadenylation sequences are provided by the 3'-end sequences of any of the highly expressed genes, like ADH1, MFα1, or TPI (Alber, T. and Kawasaki, G., J. of Mol. & Appl. Genet. 1:419-434, 1982).

A number of yeast expression plasmids like YEp6, YEp13, YEp24 can be used as vectors. A gene of interest such as HRSV-like protein cDNA can be fused to any of the promoters mentioned above, and then ligated to the plasmids for expression in various yeast hosts. The above-mentioned plasmids have been fully described in the literature (Botstein, et al., Gene, 8:17-24, 1979; Broach, et al., Gene, 8:121-133, 1979).

Two procedures are used in transforming yeast cells. In one case, yeast cells are first converted into protoplasts using zymolyase, lyticase or glusulase, followed by addition of DNA and polyethylene glycol (PEG). The PEG-treated protoplasts are then regenerated in a 3% agar medium under selective conditions. Details of this procedure are given in the papers by J.D. Beggs, Nature (London), 275:104-109 (1978); and Hinnen, A., et al., Proc. Natl.-Acad. Sci. USA, 75:1929-1933 (1978). The second procedure does not involve removal of the cell wall. Instead the cells are treated with lithium-chloride or acetate and PEG and put on selective plates (Ito, H., et al., J. Bact., 153:163-168, 1983).

HRSV-like proteins can be isolated from yeast by lysing the cells and applying standard protein isolation techniques to the lysates. The proteins can be detected by using Western blot techniques or radioimmunoassays.

### E. Expression in Cell Cultures.

The HRSV cDNA can be ligated to various expression vectors for use in transforming host cell cultures. The vectors all contain gene sequences to initiate transcription and translation of the HRSV-like proteins that are compatible with the host cell to be transformed.

In addition, the vectors preferably contain a marker to provide a phenotypic trait for selection of transformed host cells such as dihydrofolate reductase or metallothionein. Additionally a replicating vector might contain a replicon.

Illustrative of cell cultures useful for the production of HRSV-like proteins are cells of insect or mammalian origin. Mammalian cell systems often will be in the form of monolayers of cells although mammalian cell suspensions may also be used. Illustrative examples of mammalian cell lines include VERO and HeLa cells, Chinese hamster ovary (CHO) cell lines, WI38, BHK, COS-7 or MDCK cell lines.

As indicated above, the vector, e.g., a plasmid, which is used to transform the host cell preferably contains gene sequences to initiate the transcription and translation of the HRSV-like proteins gene sequence. These sequences are referred to as expression control sequences. When the host cell is of mammalian or insect origin illustrative useful expression control sequences are obtained from the SV-40 promoter (Science, 222, 524-527, 1983), the CMV I.E. promoter (Proc. Natl. Acad. Sci. 81:659-663, 1984), the metallothionein promoter (Nature, 296, 39-42, 1982) or the baculovirus polyhedrin promoter (insect cells) (Virol., 131, 561-565, 1983). The plasmid or replicating or integrating DNA material containing the expression control sequences is cleaved using restriction enzymes and adjusted in size as necessary or desirable and ligated with cDNA coding for HRSV-like proteins by means well known in the art.

As with yeast when higher animal host cells are employed, polyadenylation or transcription terminator sequences from known mammalian genes need to be incorporated into the vector. An example of a terminator sequence is the polyadenylation sequence from the bovine growth hormone gene.

The HRSV glycoprotein F may be designed to be secreted from cells into the surrounding media. This is accomplished by causing the early termination of the glycoprotein prior to its anchor region. L. Lasky, et al., Biotechnology, 2:527-532 (1984). The anchor is a hydrophobic region at the carboxy terminal end of the glycoprotein which causes the retention of the glycoprotein in the cell membrane. Early termination may be accomplished by inserting a universal translational terminator oligonucleotide into an appropriate site in the gene's DNA. These oligonucleotides are commercially available. For the F gene, a preferred site for insertion is the NsiI restriction enzyme site which is approximately 1.5 kb from the 5' end of the gene.

Additionally gene sequences to control replication in the host cell may be incorporated into the vector such as those found in bovine papillomavirus type-vectors. Saveria-Campo, M., "Bovine papillomavirus DNA: a eukaryotic cloning vector" in DNA Cloning Vol II a practical approach. Ed. D.M. Glover, IRL Press, Arlington, Virginia pages 213-238 (1985).

The preferred expression vector useful for expressing HRSV-like proteins in Chinese hamster ovary (CHO) cell is a shuttle vector pSVCOW7 which replicates in both CHO and E. coli cells utilizing ampicillin resistance and dihydrofolate reductase genes as markers in E. coli and CHO cells respectively. Plasmid pSVCOW7 also provides the polyadenylation sequence from bovine growth hormone which is necessary for expression in CHO cells. Plasmid pSVCOW7 is cleaved and a viral promoter and the HRSV-like protein cDNAs inserted.

The preferred expression vector useful in forming recombinant baculovirus for expressing HRSV-like proteins in insect cells is pAc373. Smith et al., Mol. Cell. Biol. 3:2156-2165 (1983). The plasmid replicates in E. coli cells utilizing ampicillin resistance, and provides the eukaryotic promoter and polyadenylation signal from the baculovirus polyhedrin gene for expression of HRSV genes. Plasmid pAc373 is cleaved and a HRSV cDNA is inserted adjacent to the promoter. This new plasmid is cotransfected with baculovirus (Autograpa californica nuclear polyhedrosis virus) DNA into insect cells by calcium phosphate precipitation. Recombinant baculovirus in which the pAc373 polyhedrin gene containing a HRSV cDNA has replaced the resident viral polyhedrin gene by homologous recombination is detected by dot blot hybridization (Summers, M., and G, Smith, A manual of methods for baculovirus vectors and insect cell culture procedures, Texas A & M University, College Station, Texas, pp. 29-30 (1986)) using ³²p-labeled HRSV cDNA as a probe. Insect cells infected with recombinant baculovirus may also be differentiated by their inclusion-negative morphology since the insertion of the HRSV cDNA into the polyhedrin gene prevents the synthesis of this inclusion-forming protein. Isolation of HRSV proteins from infected insect cells is accomplished as described for CHO cells.

The preferred expression vector used in conjunction with bovine papilloma virus (BPV) for expressing HRSV-like proteins is pTFW9 U.S. Serial No. 935,490, which is incorporated by reference herein. The plasmid replicates in E. coli utilizing ampicillin resistance, and provides the mouse metallothionein promoter and SV40 polyadenylation signal for expression of HRSV genes. Plasmid pTFW9 is cleaved and a HRSV cDNA is inserted adjacent to the promoter. This new plasmid is then cleaved to allow insertion of BPV. The recombinant plasmid is transfected into animal cells by calcium phosphate precipitation and foci of transformed cells are selected. HRSV protein expressed in these transformed cells is isolated as described for CHO cells.

The host cells are competent or rendered competent for transfection by various means. There are several well-known methods of introducing DNA into animal cells. These include: calcium phosphate precipitation, fusion of the recipient cells with bacterial protoplasts containing the DNA, treatment of the recipient cells with liposomes containing the DNA, and microinjection of the DNA directly into the cells.

The transfected cells are cultured by means well known in the art. Biochemical Methods in Cell Culture and Virology, Kuchler, R. J., Dowden, Hutchinson and Ross, Inc., (1977). and the expressed HRSV-like proteins analogs are isolated from cell suspensions created by disruption of the host cell system by well known mechanical or enzymatic means. HRSV-like proteins which are designed to be secreted from the cells are isolated from the media without disruption of the cells.

Isolation of the HRSV proteins is accomplished by lysing the CHO cells with detergents. For HRSV glycoproteins it is helpful to first apply the cytoplasmic fraction to a lentil lectin column which will specifically bind glycoproteins. The eluted glycoproteins are then applied to an affinity column containing anti-HRSV antibody. Non-glycoproteins of HRSV can be directly applied to the affinity column.

### F. Definitions.

The phrase "cell culture" refers to the containment of growing cells derived from either a multicellular plant or animal which allows for the cells to remain viable outside the original plant or animal.

The term "downstream" identifies sequences proceeding farther in the direction of expression; for example, the coding region is downstream from the initiation codon.

The term "microorganism" includes both single cellular prokaryote and eukaryote organisms such as bacteria, actinomycetes and yeast.

The term "operon" is a complete unit of gene expression and regulation, including structural genes, regulator genes and control elements in DNA recognized by regulator gene product.

The term "plasmid" refers to an autonomous self-replicating extrachromosomal circular DNA and includes both the expression and nonexpression types. Where a recombinant microorganism or cell culture is described as hosting an expression plasmid the phrase "expression plasmid" includes both extrachromosomal circular DNA and DNA that has been incorporated into the host chromosome(s). Where a plasmid is being maintained by a host cell, the plasmid is either being stably replicated by the cells during mitosis as an autonomous structure or as an incorporated portion of the host's genome.

The term "promoter" is a region of DNA involved in binding the RNA polymerase to initiate transcription.

The phrase "immunogenic fragments" includes derivatives of the structural proteins of HRSV having sufficient antigenic capacity to produce effective immunologic protection in patient exposed to virulent HRSV. The phrase "HRSV-like proteins" is meant to encompass these fragments. For example, HRSV proteins are made up of amino acid residues, not all of which are exposed to the aqueous environment and capable of eliciting a strong immunogenic response. If carefully selected, modification or deletion to these regions would not affect antigenicity. While no longer being native HRSV proteins, the proteins are now immunogenic fragments if deletions are involved and HRSV-like proteins if either deletions or modifications to the primary sequence were involved.

The phrase "DNA sequence" refers to a single or double stranded DNA molecule comprised of nucleotide bases, adenosine, thymidine, cytosine and guanosine.

The phrase "essentially pure (HRSV) protein" refers to compositions of viral protein that contain no virus synthesized protein. Although the essentially pure proteins may be contaminated with low levels of host cell constituents, the protein is devoid of contaminating structural and non-structural viral protein produced by replicating HRSV.

The phrase "suitable host" refers to a cell culture or microorganism that is compatible with a recombinant plasmid and will permit the plasmid to replicate, to be incorporated into its genome or to be expressed.

The term "upstream" identifies sequences proceeding in the opposite direction from expression; for example, the bacterial promoter is upstream from the transcription unit, the initiation codon is upstream from the coding region.

### Example 1 Preparation of a Vaccine for HRSV

The immunogen can be prepared in vaccine dose form by well-known procedures. The vaccine can be administered intramuscularly, subcutaneously or intranasally. For parenteral administration, such as intramuscular injection, the immunogen may be combined with a suitable carrier, for example, it may be administered in water, saline or buffered vehicles with or without various adjuvants or immunomodulating agents such as aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, Corynebacterium parvum (Propionobacterium acnes), Bordetella pertussis, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.).

The proportion of immunogen and adjuvant can be varied over a broad range so long as both are present in effective amounts. For example, aluminum hydroxide can be present in an amount of about 0.5% of the vaccine mixture (Al₂O₃ basis). On a per dose basis, the concentration of the immunogen can range from about 0.015 µg to about 1.5 mg per kilogram per patient body weight. A preferable dosage range is from about 1.5 µg/kg to about .043 mg/kg of patient body weight. A suitable dose size in humans is about 0.1 - 1 ml, preferably about 0.1 ml. Accordingly, a dose for intramuscular injection, for example, would comprise 0.1 ml containing immunogen in admixture with 0.5% aluminum hydroxide.

The vaccine can be administered to pregnant women or to women of child-bearing age to stimulate maternal HRSV antibodies. The female can be revaccinated as needed. Infants can be vaccinated at 2 to 3 months of age and revaccinated as necessary, preferably at 6 to 9 months of age. Babies born to unvaccinated mothers can be vaccinated at 2 to 3 months of age. The vaccine may also be useful in other susceptible populations such as elderly or infirmed patients.

The vaccine may also be combined with other vaccines for other diseases to produce multivalent vaccines. It may also be combined with other medicaments such as antibiotics.

CHART 1

Nucleotide sequence of the F mRNA
and the predicted protein sequence

G GGG CAA ATA ACA ATG GAG TTG CTA ATC CTC AAA 34
Met Glu Leu Leu Ile Leu Lys 7

GCA AAT GCA ATT ACC ACA ATC CTC ACT GCA GTC ACA TTT TGT TTT GCT TCT GGT 88
Ala Asn Ala Ile Thr Thr Ile Leu Thr Ala Val Thr Phe Cys Phe Ala Ser Gly 25

CAA AAC ATC ACT GAA GAA TTT TAT CAA TCA ACA TGC AGT GCA GTT AGC AAA GGC 142
Gln Asn Ile Thr Glu Glu Phe Tyr Gln Ser Thr Cys Ser Ala Val Ser Lys Gly 43

TAT CTT AGT GCT CTG AGA ACT GGT TGG TAT ACC AGT GTT ATA ACT ATA GAA TTA 196
Tyr Leu Ser Ala Leu Arg Thr Gly Trp Tyr Thr Ser Val Ile Thr Ile Glu Leu 61

AGT AAT ATC AAG GAA AAT AAG TGT AAT GGA ACA GAT GCT AAG GTA AAA TTG ATA 250
Ser Asn Ile Lys Glu Asn Lys Cys Asn Gly Thr Asp Ala Lys Val Lys Leu Ile 79

AAA CAA GAA TTA GAT AAA TAT AAA AAT GCT GTA ACA GAA TTG CAG TTG CTC ATG 304
Lys Gln Glu Leu Asp Lys Tyr Lys Asn Ala Val Thr Glu Leu Gln Leu Leu Met 97

CAA AGC ACA CCA CCA ACA AAC AAT CGA GCC AGA AGA GAA CTA CCA AGG TTT ATG 358
Gln Ser Thr Pro Pro Thr Asn Asn Arg Ala Arg Arg Glu Leu Pro Arg Phe Met 115

AAT TAT ACA CTC AAC AAT GCC AAA AAA ACC AAT GTA ACA TTA AGC AAG AAA AGG 412
Asn Tyr Thr Leu Asn Asn Ala Lys Lys Thr Asn Val Thr Leu Ser Lys Lys Arg 133

AAA AGA AGA TTT CTT GGT TTT TTG TTA GGT GTT GGA TCT GCA ATC GCC AGT GGC 466
Lys Arg Arg Phe Leu Gly Phe Leu Leu Gly Val Gly Ser Ala Ile Ala Ser Gly 151

GTT GCT GTA TCT AAG GTC CTG CAC CTA GAA GGG GAA GTG AAC AAG ATC AAA AGT 520
Val Ala Val Ser Lys Val Leu His Leu Glu Gly Glu Val Asn Lys Ile Lys Ser 169

CHART 1 (Continued)

GCT CTA CTA TCC ACA AAC AAG GCT GTA GTC AGC TTA TCA AAT GGA GTT AGT GTC 574
Ala Leu Leu Ser Thr Asn Lys Ala Val Val Ser Leu Ser Asn Gly Val Ser Val 187

TTA ACC AGC AAA GTG TTA GAC CTC AAA AAC TAT ATA GAT AAA CAA TTG TTA CCT 628
Leu Thr Ser Lys Val Leu Asp Leu Lys Asn Tyr Ile Asp Lys Gln Leu Leu Pro 205

ATT GTG AAC AAG CAA AGC TGC AGC ATA TCA AAT ATA GAA ACT GTG ATA GAG TTC 682
Ile Val Asn Lys Gln Ser Cys Ser Ile Ser Asn Ile Glu Thr Val Ile Glu Phe 223

CAA CAA AAG AAC AAC AGA CTA CTA GAG ATT ACC AGG GAA TTT AGT GTT AAT GCA 736
Gln Gln Lys Asn Asn Arg Leu Leu Glu Ile Thr Arg Glu Phe Ser Val Asn Ala 241

GGT GTA ACT ACA CCT GTA AGC ACT TAC ATG TTA ACT AAT AGT GAA TTA TTG TCA 790
Gly Val Thr Thr Pro Val Ser Thr Tyr Met Leu Thr Asn Ser Glu Leu Leu Ser 259

TTA ATC AAT GAT ATG CCT ATA ACA AAT GAT CAG AAA AAG TTA ATG TCC AAC AAT 844
Leu Ile Asn Asp Met Pro Ile Thr Asn Asp Gln Lys Lys Leu Met Ser Asn Asn 277

GTT CAA ATA GTT AGA CAG CAA AGT TAC TCT ATC ATG TCC ATA ATA AAA GAG GAA 898
Val Gln Ile Val Arg Gln Gln Ser Tyr Ser Ile Met Ser Ile Ile Lys Glu Glu 295

GTC TTA GCA TAT GTA GTA CAA TTA CCA CTA TAT GGT GTT ATA GAT ACA CCC TGT 952
Val Leu Ala Tyr Val Val Gln Leu Pro Leu Tyr Gly Val Ile Asp Thr Pro Cys 313

TGG AAA CTA CAC ACA TCC CCT CTA TGT ACA ACC AAC ACA AAA GAA GGG TCC AAC 1006
Trp Lys Leu His Thr Ser Pro Leu Cys Thr Thr Asn Thr Lys Glu Gly Ser Asn 331

ATC TGT TTA ACA AGA ACT GAC AGA GGA TGG TAC TGT GAC AAT GCA GGA TCA GTA 1060
Ile Cyc Leu Thr Arg Thr Asp Arg Gly Trp Tyr Cys Asp Asn Ala Gly Ser Val 349

TCT TTC TTC CCA CAA GCT GAA ACA TGT AAA GTT CAA TCA AAT CGA GTA TTT TGT 1114
Ser Phe Phe Pro Gln Ala Glu Thr Cys Lys Val Gln Ser Asn Arg Val Phe Cys 367

CHART 1 (Continued)

GAC ACA ATG AAC AGT TTA ACA TTA CCA AGT GAA ATA AAT CTC TGC AAT GTT GAC 1168
Asp Thr Met Asn Ser Leu Thr Leu Pro Ser Glu Ile Asn Leu Cys Asn Val Asp 385

ATA TTC AAC CCC AAA TAT GAT TGT AAA ATT ATG ACT TCA AAA ACA GAT GTA AGC 1222
Ile Phe Asn Pro Lys Tyr Asp Cys Lys Ile Met Thr Ser Lys Thr Asp Val Ser 403

AGC TCC GTT ATC ACA TCT CTA GGA GCC ATT GTG TCA TGC TAT GGC AAA ACT AAA 1276
Ser Ser Val Ile Thr Ser Leu Gly Ala Ile Val Ser Cys Tyr Gly Lys Thr Lys 421

TGT ACA GCA TCC AAT AAA AAT CGT GGA ATC ATA AAG ACA TTT TCT AAC GGG TGC 1330
Cys Thr Ala Ser Asn Lys Asn Arg Gly Ile Ile Lys Thr Phe Ser Asn Gly Cys 439

GAT TAT GTA TCA AAT AAA GGG ATG GAC ACT GTG TCT GTA GGT AAC ACA TTA TAT 1384
Asp Tyr Val Ser Asn Lys Gly Met Asp Thr Val Ser Val Gly Asn Thr Leu Tyr 457

TAT GTA AAT AAG CAA GAA GGT AAA AGT CTC TAT GTA AAA GGT GAA CCA ATA ATA 1438
Tyr Val Asn Lys Gln Glu Gly Lys Ser Leu Tyr Val Lys Gly Glu Pro Ile Ile 475

AAT TTC TAT GAC CCA TTA GTA TTC CCC TCT GAT GAA TTT GAT GCA TCA ATA TCT 1492
Asn Phe Tyr Asp Pro Leu Val Phe Pro Ser Asp Glu Phe Asp Ala Ser Ile Ser 493

CAA GTC AAC GAG AAG ATT AAC CAG AGC CTA GCA TTT ATT CGT AAA TCC GAT GAA 1546
Gln Val Asn Glu Lys Ile Asn Gln Ser Leu Ala Phe Ile Arg Lys Ser Asp Glu 511

TTA TTA CAT AAT GTA AAT GCT GGT AAA TCC ACC ACA AAT ATC ATG ATA ACT ACT 1600
Leu Leu His Asn Val Asn Ala Gly Lys Ser Thr Thr Asn Ile Met Ile Thr Thr 529

ATA ATT ATA GTG ATT ATA GTA ATA TTG TTA TCA TTA ATT GCT GTT GGA CTG CTC 1654
Ile Ile Ile Val Ile Ile Val Ile Leu Leu Ser Leu Ile Ala Val Gly Leu Leu 547

TTA TAC TGT AAG GCC AGA AGC ACA CCA GTC ACA CTA AGC AAA GAT CAA CTG AGT 1708
Leu Tyr Cys Lys Ala Arg Ser Thr Pro Val Thr Leu Ser Lys Asp Gln Leu Ser 565

CHART 1 (Continued)

GGT ATA AAT AAT ATT GCA TTT AGT AAC TAA ATA AAA ATA GCA CCT AAT CAT GTT 1762
Gly Ile Asn Asn Ile Ala Phe Ser Asn 574

CTT ACA ATG GTT TAC TAT CTG CTC ATA GAC AAC CCA TCT GTC ATT GGA TTT TCT 1816

TAA AAT CTG AAC TTC ATC GAA ACT CTC ATC TAT AAA CCA TCT CAC TTA CAC TAT 1870

TTA AGT AGA TTC CTA GTT TAT AGT TAT AT 1879

CHART 2

Nucleotide sequence of the RS virus G mRNA
and the predicted protein sequence

1 GGG GCA AAT GCA AAC ATG TCC AAA AAC AAG GAC CAA CGC ACC GCT AAG ACA TTA
Met Ser Lys Asn Lys Asp Gln Arg Thr Ala Lys Thr Leu

55 GAA AGG ACC TGG GAC ACT CTC AAT CAT TTA TTA TTC ATA TCA TCG TGC TTA TAT
Glu Arg Thr Trp Asp Thr Leu Asn His Leu Leu Phe Ile Ser Ser Cys Leu Tyr

109 AAG TTA AAT CTT AAA TCT GTA GCA CAA ATC ACA TTA TCC ATT CTG GCA ATG ATA
Lys Leu Asn Leu Lys Ser Val Ala Gln Ile Thr Leu Ser Ile Leu Ala Met Ile

163 ATC TCA ACT TCA CTT ATA ATT GCA GCC ATC ATA TTC ATA GCC TCG GCA AAC CAC
Ile Ser Thr Ser Leu Ile Ile Ala Ala Ile Ile Phe Ile Ala Ser Ala Asn His

217 AAA GTC ACA CCA ACA ACT GCA ATC ATA CAA GAT GCA ACA AGC CAG ATC AAG AAC
Lys Val Thr Pro Thr Thr Ala Ile Ile Gln Asp Ala Thr Ser Gln Ile Lys Asn

271 ACA ACC CCA ACA TAC CTC ACC CAG AAT CCT CAG CTT GGA ATC AGT CCC TCT AAT
Thr Thr Pro Thr Tyr Leu Thr Gln Asn Pro Gln Leu Gly Ile Ser Pro Ser Asn

325 CCG TCT GAA ATT ACA TCA CAA ATC ACC ACC ATA CTA GCT TCA ACA ACA CCA GGA
Pro Ser Glu Ile Thr Ser Gln Ile Thr Thr Ile Leu Ala Ser Thr Thr Pro Gly

379 GTC AAG TCA ACC CTG CAA TCC ACA ACA GTC AAG ACC AAA AAC ACA ACA ACA ACT
Val Lys Ser Thr Leu Gln Ser Thr Thr Val Lys Thr Lys Asn Thr Thr Thr Thr

433 CAA ACA CAA CCC AGC AAG CCC ACC ACA AAA CAA CGC CAA AAC AAA CCA CCA AGC
Gln Thr Gln Pro Ser Lys Pro Thr Thr Lys Gln Arg Gln Asn Lys Pro Pro Ser

487 AAA CCC AAT AAT GAT TTT CAC TTT GAA GTG TTC AAC TTT GTA CCC TGC AGC ATA
Lys Pro Asn Asn Asp Phe His Phe Glu Val Phe Asn Phe Val Pro Cys Ser Ile

CHART 2 (Continued)

541 TGC AGC AAC AAT CCA ACC TGC TGG GCT ATC TGC AAA AGA ATA CCA AAC AAA AAA
Cys Ser Asn Asn Pro Thr Cys Trp Ala Ile Cys Lys Arg Ile Pro Asn Lys Lys

595 CCA GGA AAG AAA ACC ACT ACC AAG CCC ACA AAA AAA CCA ACC CTC AAG ACA ACC
Pro Gly Lys Lys Thr Thr Thr Lys Pro Thr Lys Lys Pro Thr Leu Lys Thr Thr

649 AAA AAA GAT CCC AAA CCT CAA ACC ACT AAA TCA AAG GAA GTA CCC ACC ACC AAG
Lys Lys Asp Pro Lys Pro Gln Thr Thr Lys Ser Lys Glu Val Pro Thr Thr Lys

703 CCC ACA GAA GAG CCA ACC ATC AAC ACC ACC AAA ACA AAC ATC ATA ACT ACA CTA
Pro Thr Glu Glu Pro Thr Ile Asn Thr Thr Lys Thr Asn Ile Ile Thr Thr Leu

757 CTC ACC TCC AAC ACC ACA GGA AAT CCA GAA CTC ACA AGT CAA ATG GAA ACC TTC
Leu Thr Ser Asn Thr Thr Gly Asn Pro Glu Leu Thr Ser Gln Met Glu Thr Phe

811 CAC TCA ACT TCC TCC GAA GGC AAT CCA AGC CCT TCT CAA GTC TCT ACA ACA TCC
His Ser Thr Ser Ser Glu Gly Asn Pro Ser Pro Ser Gln Val Ser Thr Thr Ser

865 GAG TAC CCA TCA CAA CCT TCA TCT CCA CCC AAC ACA CCA CGC CAG TAG TTA CTT
Glu Tyr Pro Ser Gln Pro Ser Ser Pro Pro Asn Thr Pro Arg Gln End

919 AAA AAA AAA AAA AAA AA 935

## Claims (Claims for the following Contracting State(s): BE, CH, DE, FR, GB, IT, LU, NL, SE)

1. A human respiratory syncytial virus vaccine comprising glycosylated F protein, glycosylated G protein or a derivative thereof having sufficient antigenic capacity to produce effective immunologic protection in a patient exposed to virulent HRSV, free of other virus-synthesised protein.

2. A vaccine according to claim 1, comprising glycosylated F protein or an immunogenic fragment thereof.

3. A vaccine according to claim 1, comprising glycosylated G protein or an immunogenic fragment thereof.

4. Use of a glycosylated protein as defined in any of claims 1 to 3, free of other virus-synthesised protein, for the preparation of a medicament to protect humans from human respiratory syncytial virus.

## Claims (Claims for the following Contracting State(s): AT)

1. A method for preparing a human respiratory syncytial virus vaccine, which comprises formulating glycosylated F protein, glycosylated G protein or a derivative thereof having sufficient antigenic capacity to produce effective immunologic protection in a patient exposed to virulent HRSV, free of other virus-synthesised protein with a suitable carrier.

2. A method according to claim 1, wherein the vaccine comprises glycosylated F protein or an immunogenic fragment thereof.

3. A method according to claim 1, wherein the vaccine comprises glycosylated G protein or an immunogenic fragment thereof.

## Patentansprüche (Patentansprüche für folgende(n) Vertragsstaat(en): BE, CH, DE, FR, GB, IT, LU, NL, SE)

1. Impfstoff gegen menschliches respiratorisches Syncytialvirus, enthaltend glycosyliertes F-Protein, glycosyliertes G-Protein oder ein Derivat davon mit ausreichender antigener Kapazität, um in einem Patienten, der virulentem HRSV ausgesetzt ist, wirksamen immunologischen Schutz zu erzeugen, das frei von anderem virussynthetisiertem Protein ist.

2. Impfstoff nach Anspruch 1, enthaltend glycosyliertes F-Protein oder ein immunogenes Fragment davon.

3. Impfstoff nach Anspruch 1, enthaltend glycosyliertes G-Protein oder ein immunogenes Fragment davon.

4. Verwendung eines glycosylierten Proteins, wie in einem der Ansprüche 1 bis 3 definiert, das frei von anderem virussynthetisiertem Protein ist, für die Herstellung eines Medikamentes zum Schutz von Menschen vor menschlichem respiratorischem Syncytialvirus.

## Patentansprüche (Patentansprüche für folgende(n) Vertragsstaat(en): AT)

1. Verfahren zum Herstellen eines Impfstoffs gegen menschliches respiratorisches Syncytialvirus, umfassend das Zubereiten von glycosyliertem F-Protein, glycosyliertem G-Protein oder einem Derivat davon mit ausreichender antigener Kapazität, um in einem Patienten, der virulentem HRSV ausgesetzt ist, wirksamen immunologischen Schutz zu erzeugen, das frei von anderem virussynthetisiertem Protein ist, zusammen mit einem geeigneten Träger.

2. Verfahren nach Anspruch 1, worin der Impfstoff glycosyliertes F-Protein oder ein immunogenes Fragment davon enthält.

3. Verfahren nach Anspruch 1, worin der Impfstoff glycosyliertes G-Protein oder ein immunogenes Fragment davon enthält.

## Revendications (Revendications pour l'(les) Etat(s) contractant(s) suivant(s): BE, CH, DE, FR, GB, IT, LU, NL, SE)

1. Vaccin contre le virus syncytial respiratoire humain comprenant une protéine F glycosylée, une protéine G glycosylée ou un de ses dérivés ayant une capacité antigénique suffisante pour conférer une protection immunologique efficace à un patient mis en contact avec un HRSV virulent, dépourvu d'une autre protéine synthétisèe par un virus.

2. Vaccin suivant la revendication 1, comprenant une protéine F glycosylée ou un de ses fragments immunogènes.

3. Vaccin suivant la revendication 1, comprenant une protéine G glycosylée ou un de ses fragments immunogènes.

4. Utilisation d'une protéine glycosylée suivant l'une quelconque des revendications 1 à 3, dépourvue d'une autre protéine synthétisée par un virus, pour la préparation d'un médicament destiné à protéger l'homme contre le virus syncytial respiratoire humain.

## Revendications (Revendications pour l'(les) Etat(s) contractant(s) suivant(s): AT)

1. Procédé de préparation d'un vaccin contre le virus syncytial respiratoire humain, qui comprend la formulation d'une protéine F glycosylée, d'une protéine G glycosylée ou d'un de ses dérivés ayant une capacité antigénique suffisante pour conférer une protection immunologique efficace à un patient mis en contact avec un HRSV virulent, dépourvu d'une autre protéine synthétisée par un virus, avec un support convenable.

2. Procédé suivant la revendication 1, dans lequel le vaccin comprend une protéine F glycosylée ou un de ses fragments immunogènes.

3. Procédé suivant la revendication 1, dans lequel le vaccin comprend une protéine G glycosylée ou un de ses fragments immunogènes.
